# EUROPEAN PATENT APPLICATION

(11) **EP 0 581 464 A1**
(43) Date of publication of application: **02.02.1994**
(21) Application number: 93305379.5
(22) Date of filing: 08.07.1993
(51) Int. Cl.: C07D 309/10, C07D 405/12, A61K 31/35

(54) **Ester derivatives and pharmaceutical compositions containing them**

(30) Priority: 15.07.1992 EP 92402035; 16.03.1993 EP 93400672
(71) Applicant: ZENECA LIMITED, London W1Y 6LN (GB); ZENECA Pharma S.A., F-95022 Cergy Pontoise Cédex (FR)
(72) Inventor: Bird, Thomas Geoffrey Colerick, F-51064 Reims Cedex (FR); Olivier, Annie Antoinette Christiane, F-51064 Reims Cedex (FR)
(74) Representative: Tait, Brian Steele

(57) **Abstract**

The invention concerns ester derivatives of the formula I 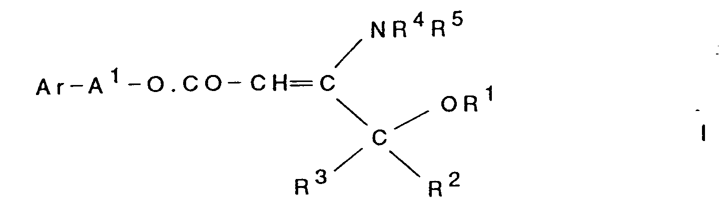
wherein Ar is phenyl, naphthyl, indenyl, indanyl, a 10-membered bicyclic heterocyclic moiety containing one or two nitrogen heteroatoms and optionally containing a further heteroatom selected from nitrogen, oxygen and sulphur, or a 9-membered or 10-membered bicyclic heterocyclic moiety containing one heteroatom group selected from oxygen, sulphur, sulphinyl, sulphonyl and imino;
A¹ is a direct link to the oxygen atom of the ester group or A¹ is (1-3C)alkylene ;
each of R⁴ and R⁵ is hydrogen or (1-4C)alkyl;
R¹ is (1-4C)alkyl, (3-4C)alkenyl or (3-4C)alkynyl ; and
R² and R³ together form a group of the formula -A²-X-A³- wherein each of A² and A3 is independently (1-3C)alkylene and X is oxy, thio, sulphinyl, sulphonyl or imino;
or pharmaceutically-acceptable salts thereof ;
processes for their manufacture; pharmaceutical compositions containing them and their use as 5-lipoxygenase inhibitors.

## Description

This invention concerns ester derivatives and more particularly ester derivatives which are inhibitors of the enzyme 5-lipoxygenase (hereinafter referred to as 5-LO). The invention also concerns processes for the manufacture of said ester derivatives and novel pharmaceutical compositions containing them. Also included in the invention is the use of said ester derivatives in the treatment of various inflammatory and/or allergic diseases in which the direct or indirect products of 5-LO catalysed oxidation of arachidonic acid are involved, and the production of new medicaments for such use.

As stated above the ester derivatives described hereinafter are inhibitors of 5-LO, which enzyme is known to be involved in catalysing the oxidation of arachidonic acid to give rise via a cascade process to the physiologically active leukotrienes such as leukotriene B₄ (LTB₄) and the peptido-lipid leukotrienes such as leukotriene C₄ (LTC₄) and leukotriene D₄ (LTD₄) and various metabolites.

The biosynthetic relationship and physiological properties of the leukotrienes are summarised by G.W. Tay- lor and S.R. Clarke in Trends in Pharmacological Sciences, 1986, 7, 100-103. The leukotrienes and their metabolites have been implicated in the production and development of various inflammatory and allergic diseases such as inflammation of thejoints (especially rheumatoid arthritis, osteoarthritis and gout), inflammation of the gastrointestinal tract (especially inflammatory bowel disease, ulcerative colitis and gastritis), skin disease (especially psoriasis, eczema and dermatitis) and respiratory disease (especially asthma, bronchitis and allergic rhinitis), and in the production and development of various cardiovascular and cerebrovascular disorders such as myocardial infarction, angina and peripheral vascular disease. In addition the leukotrienes are mediators of inflammatory diseases by virtue of their ability to modulate lymphocyte and leukocyte function. Other physiologically active metabolites of arachidonic acid, such as the prostaglandins and thromboxanes, arise via the action of the enzyme cyclooxygenase on arachidonic acid.

It is disclosed in European Patent Application Nos 0375404 and 0385662 that certain heterocyclic derivatives possess inhibitory properties against 5-LO. Furthermore European Patent Applications Nos. 0409413, 0420511, 0462812, 0462813, 0466452 and 0488602 are also concerned with heterocyclic derivatives which possess inhibitory properties against 5-LO. We have now discovered that certain ester derivatives, which possess some structural features which are similar to those of the compounds disclosed in the above-mentioned applications but which possess other structural features in particular an ester group which was not envisaged in those earlier applications, are effective inhibitors of the enzyme 5-LO and thus of leukotriene biosyntheses. Thus such compounds are of value as therapeutic agents in the treatment of, for example, allergic conditions, psoriasis, asthma, cardiovascular and cerebrovascular disorders, and/or inflammatory and arthritic conditions, mediated alone or in part by one or more leukotrienes.

According to the invention there is provided an ester derivative of the formula I (set out hereinafter) wherein Ar is phenyl, naphthyl, indenyl or indanyl, or a 10-membered bicyclic heterocyclic moiety containing one or two nitrogen heteroatoms and optionally containing a further heteroatom selected from nitrogen, oxygen and sulphur, or a 9-membered or 10-membered bicyclic heterocyclic moiety containing one heteroatom group selected from oxygen, sulphur, sulphinyl, sulphonyl and imino, and Ar may optionally bear up to four substituents selected from halogeno, hydroxy, amino, cyano, formyl, oxo, thioxo, (1-4C)alkyl, (1-4C)alkoxy, fluoro-(1-4C)alkyl, hydroxy-(1-4C)alkyl, (2-4C)alkanoyl, (2-4C)alkanoylamino, N-(1-4C)alkyl-(2-4C)alkanoylamino, hydroxyimino-(1-4C)alkyl, (1-4C)alkoxyimino-(1-4C)alkyl, (2-5C)alkanoyloxyimino-(1-4C)alkyl, cyano-(1-4C)alkoxyi- mino-(1-4C)alkyl, hydroxyamino(1-4C)alkyl, (1-4C)alkoxyamino-(1-4C)alkyl, _N-hydroxyureido-(1-4C)alkyl, N-(1-4C)alkoxyureido-(1-4C)alkyl, N-hydroxy-(2-4C)alkanoylamino-(1-4C)alkyl, N-(1-4C)alkoxy-(2-4C)-alkanoylamino-(1-4C)alkyl, (1-6C)alkylideneaminooxy-(1-4C)alkyl, (1-4C)alkanesulphonamido, N-(1-4C)alkyl-(1-4C)alkanesulphonamido, N-(1-4C)alkylsulphamoyl, N,N-di-[(1-4C)alkyl]sulphamoyl, phenyl, benzoyl, benzyl, N-phenylsulphamoyl, N-(1-4C)alkyl-N-phenylsulphamoyl, hydroxyimino, (1-4C)alkoxyimino, (2-5C)alkanoy- loxyimino and cyano-(1-4C)alkoxyimino, and wherein said phenyl substituent or any of said substituents which contains a phenyl group may optionally bear a substituent selected from halogeno, (1-4C)alkyl and (1-4C)al- koxy;
A¹ is a direct link to the oxygen atom of the ester group or A is (1-3C) alkylene;
R⁴ is hydrogen or (1-4C)alkyl;
R⁵ is hydrogen or (1-4C)alkyl;
R¹ is (1-4C)alkyl, (3-4C)alkenyl or (3-4C)alkynyl; and
R² and R³ together form a group of the formula -A²⁻X-A^{3 -} which together with the carbon atom to which A² and A3 are attached define a ring having 5 or 6 ring atoms, wherein each of A² and A3 is independently (1-3C)alkylene and X is oxy, thio, sulphinyl, sulphonyl or imino, and which ring may optionally bear one or two substituents selected from hydroxy, (1-4C)alkyl and (1-4C)alkoxy;
or a pharmaceutically-acceptable salt thereof.

The chemical formulae referred to herein by Roman numerals are set out for convenience on a separate sheet hereinafter.

In this specification the generic term "alkyl" includes both straight-chain and branched-chain alkyl groups. However references to individual alkyl groups such as "propyl" are specific for the straight-chain version only and references to individual branched-chain alkyl groups such as "isopropyl" are specific for the branched-chain version only. An analogous convention applies to other generic terms.

It is to be understood that, insofar as certain of the compounds of the formula I defined above may exhibit the phenomenon of tautomerism and any formula drawing presented herein may represent only one of the possible tautomeric forms, the invention includes in its definition any tautomeric form of a compound of the formula I which possesses the property of inhibiting 5-LO and is not to be limited merely to any one tautomeric form utilised within the formulae drawings.

It is further to be understood that, insofar as certain of the compounds of the formula I defined above are oxime derivatives and it is well known that oxime derivatives may exist in different geometric isomeric forms, commonly designated as (F)- or (Z)-isomers, the invention includes in its definition any such geometric isomeric form which possesses the property of inhibiting 5-LO. The separation of such geometric isomeric forms may be possible by the standard laboratory techniques of organic chemistry such as by chromatographic separation of a mixture of said isomeric forms or by crystallisation of one such isomeric form from a mixture thereof.

It is further to be understood that, insofar as certain of the compounds of formula I defined above may exist in optically active or racemic forms by virtue of one or more asymmetric carbon atoms, the invention includes in its definition any such optically active or racemic form which possesses the property of inhibiting 5-LO. The synthesis of optically active forms may be carried out by standard techniques of organic chemistry well known in the art, for example by synthesis from optically active starting materials or by resolution of a racemic form.

Suitable values for the generic terms referred to above include those set out below.

A suitable value for Ar when it is naphthyl is, for example 1-naphthyl or 2-naphthyl.

A suitable value for Ar when it is indenyl or indanyl is, for example, 5-indenyl, 6-indenyl, 5-indanyl or 6- indanyl.

A suitable value for Ar when it is a 10-membered bicyclic heterocyclic moiety containing one or two nitrogen heteroatoms and optionally containing a further heteroatom selected from nitrogen, oxygen and sulphur is, for example, a 10-membered benzo-fused heterocyclic moiety such as quinolyl, isoquinolyl, cinnolinyl, quinazolinyl, quinoxalinyl, 4H-1,4-benzoxazinyI or4H-1,4-benzothiazinyl, or a hydrogenated derivative thereof such as 1,2-dihydroquinolyl, 1,2,3,4-tetrahydroquinolyl, 1,2-dihydroisoquinolyl, 2,3-dihydro-4H-1,4-benzoxazinyl or 2,3-dihydro-4H-1,4-benzothiazinyl; or, for example, a 10-membered pyrido-fused heterocyclic moiety such as 1,7-naphthyridinyl, 1,8-naphthyridinyl, pyrido[2,3-d]pyrimidinyl, pyrido[2,3-b]pyrazinyl, 4H-pyridol3,2-b][1,4]oxazinyl and 4H-pyrido[3,2-b][1,4]thiazinyl, or a hydrogenated derivative thereof.

The heterocyclic moiety may be attached through any available position including from either of the two rings of the bicyclic heterocyclic moiety and including through an available nitrogen atom. The heterocyclic moiety may bear a suitable substituent such as, for example, a (1-4C)alkyl, fluoro-(1-4C)alkyl, phenyl, benzoyl or benzyl substituent on an available nitrogen atom.

A suitable value for Ar when it is a 9-membered or 10-membered bicyclic heterocyclic moiety containing one heteroatom group selected from oxygen, sulphur, sulphinyl, sulphonyl and imino is, for example, a 9-membered or 10-membered benzo-fused heterocyclic moiety such as benzofuranyl, benzothienyl, indolyl, 4H-chro- menyl or4H-benzothiapyranyi, or a hydrogenated derivative thereof such as 2,3-dihydrobenzofuranyl, 2,3-dihydrobenzothienyl indolinyl, chromanyl or 2,3-dihydro-4H-benzothiapyranyl.

Suitable values for substituents which may be present on Ar, on the phenyl substituent on Ar or on any of the substituents on Ar which contain a phenyl group include, for example:-
for halogeno: fluoro, chloro, bromo and iodo;
for (1-4C)alkyl: methyl, ethyl, propyl, isopropyl, butyl, isobutyl, sec-butyl and tert-butyl;
for (1-4C)alkoxy: methoxy, ethoxy, propoxy, isopropoxy and butoxy;
for fluoro-(1-4C)alkyl: fluoromethyl, difluoromethyl, trifluoromethyl, 2-fluoroethyl, 2,2,2-trifluoroethyl and pentafluoroethyl;
for hydroxy-(1-4C)alkyl: hydroxymethyl, 2-hydroxyethyl and 3-hydroxypropyl;
for (2-4C)alkanoyl: acetyl, propionyl and butyryl;
for (2-4C)alkanoylamino: acetamido, propionamido and butyramido;
for N-(1-4C)alkyl-(2-4C)-
alkanoylamino: N-methylacetamido, N-ethylacetamido and N-methylpropionamido;
for hydroxyimino-(1-4C)alkyl: hydroxyiminomethyl, 1-hydroxyiminoethyl and 2-hydroxyiminoethyl;
for (1-4C)alkoxyimino-(1-4C)alkyl: methoxyiminomethyl, ethoxyiminomethyl, 1-methoxyiminoethyl and 2-methoxyiminoethyl;
for (2-5C)alkanoyloxyimino-(1-4C)alkyl: acetoxyiminomethyl, propionyloxy- iminomethyl, 1-acetoxyiminoethyl and 2-acetoxyiminoethyl;
for cyano-(1-4C)alkoxyimino-(1-4C)alkyl: cyanomethoxyiminomethyl, 2-cyanoethoxy- iminomethyl, 1-cyanomethoxyiminoethyl and 2-cyanomethoxyiminoethyl;
for hydroxyamino-(1-4C)alkyl: hydroxyaminomethyl, 1-hydroxyaminoethyl and 2-hydroxyaminoethyl;
for (1-4C)alkoxyamino-(1-4C)alkyl: methoxyaminomethyl, ethoxyaminomethyl, 1-methoxyaminoethyl and 2-methoxyaminoethyl;
for N-hydroxyureido-(1-4C)-alkyl: N-hydroxyureidomethyl, 1-(N-hydroxy- ureido)ethyl and 2-(N-hydroxy- ureido)ethyl;
for N-(1-4C)alkoxyureido-(1-4C)alkyl: N-methoxyureidomethyl, N-ethoxyureido- methyl, 1-(N-methoxyureido)ethyl and 2-(N-methoxyureido)ethyl;
for N-hydroxy-(2-4C)alkanoylamino-(1-4C)alkyl: N-hydroxyacetamidomethyl, N-hydroxy- propionamidomethyl, 1-(N-hydroxy- acetamido)ethyl and 2-(N-hydroxy- acetamido)ethyl;
for N-(1-4C)alkoxy-(2-4C)-alkanoylamino-(1-4C)alkyl: N-methoxyacetamidomethyl, N-ethoxy- acetamidomethyl, 1-(N-methoxy- acetamido)ethyl and 2-(N-methoxy- acetamido)ethyl;
for (1-6C)alkylideneaminooxy-(1-4C)alkyl: methyleneaminooxymethyl, ethylidene- aminooxymethyl, isopropylideneamino- oxymethyl, 1-(isopropylidene- aminooxy)ethyl and 2-(isopropylidene- aminooxy)ethyl;
for (1-4C)alkanesulphonamido: methanesulphonamido and ethanesulphonamido;
for N-(1-4C)alkyl-(1-4C)-alkanesulphonamido: N-methylmethanesulphonamido, - N-ethylmethanesulphonamido and N-methylethanesulphonamido;
for N-(1-4C)alkylsulphamoyl: N-methylsulphamoyl, N-ethylsulphamoyl and N-propylsulphamoyl;
for N,N-di-[(1-4C)alkyl)-sulphamoyl: N,N-dimethylsulphamoyl, N-ethyl-N-methylsulphamoyl and N,N-diethylsulphamoyl;
for N-(1-4C)alkyl-N-phenylsulphamoyl: N-methyl-N-phenylsulphamoyl and N-ethyl-N-phenylsulphamoyl;
for (1-4C)alkoxyimino: methoxyimino and ethoxyimino;
for (2-5C)alkanoyloxyimino: acetoxyimino and propionyloxyimino;
for cyano-(1-4C)alkoxyimino: cyanomethoxyimino and 2-cyanoethoxyimino.

A suitable value for A¹ when it is (1-3C)alkylene is, for example, methylene, ethylene or trimethylene.

A suitable value for R⁴ and R⁵ when it is (1-4C)alkyl is, for example, methyl, ethyl or propyl.

A suitable value for R¹ when it is (1-4C)alkyl is, for example, methyl, ethyl, propyl or butyl; when it is (3-4C)alkenyl is, for example, allyl, 2-butenyl or 3-butenyl; and when it is (3-4C)alkynyl is, for example, 2-propynyl or 2-butynyl.

When R² and R³ together form a group of the formula -A²⁻X-A³⁻ which together with the carbon atom to which A² and A3 are attached define a ring having 5 or 6 ring atoms then a suitable value for A² or A3, when each is independently (1-3C)alkylene is, for example, methylene, ethylene or trimethylene.

Suitable values for the substituents which may be present on said 5- or 6-membered ring include, for example:-
for (1-4C)alkyl: methyl, ethyl, propyl, isopropyl, butyl and isobutyl;
for (1-4C)alkoxy: methoxy, ethoxy, propoxy, isopropoxy and butoxy.

Said substituents may be located on any available position including, when the substituent is (1-4C)alkyl, on the nitrogen atom when X is imino.

A suitable pharmaceutically-acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically-acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

Particular compounds of the invention include, for example, ester derivatives of the formula I, or pharmaceutically-acceptable salts thereof, wherein:-
(a) Ar is phenyl or naphthyl which may optionally bear one, two or three substituents selected from any of those substituents on Ar defined hereinbefore other than oxo, thioxo, hydroxyimino, (1-4C)alkoxyimino, (2-5C)alkanoyloxyimino and cyano-(1-4C)alkoxyimino; and A¹, R¹, R², R³, R⁴ and R⁵ have any of the meanings defined hereinbefore or in this section concerning particular compounds of the invention;
(b) Ar is phenyl or naphth-2-yl which may optionally bear one or two substituents selected from fluoro, chloro, methyl, ethyl, isopropyl, tert-butyl, methoxy, trifluoromethyl, phenyl, benzoyl and benzyl, and wherein said phenyl, benzoyl or benzyl substituents may optionally bear a substituent selected from fluoro, chloro, methyl and methoxy; and A^{l}, R¹, R², R³,R⁴ and R⁵ have any of the meanings defined hereinbefore or in this section concerning particular compounds of the invention;
(c) Ar is phenyl which bears one substituent selected from formyl, acetyl, propionyl, acetamido, propionamido, N-methylacetamido, N-ethylacetamido, hydroxyiminomethyl, 1-hydroxyiminoethyl, methoxyiminomethyl, ethoxyiminomethyl, 1-methoxyiminoethyl, 1-ethoxyiminoethyl, acetoxyiminomethyl, propionylox- yiminomethyl, 1-acetoxyiminoethyl, cyanomethoxyiminomethyl, 1-cyanomethoxyiminoethyl, methanesulphonamido, ethanesulphonamido, N-methylmethanesulphonamido, N-ethylmethanesulphonamido, N-methylsulphamoyl, N,N-dimethylsulphamoyl, phenyl, benzoyl, benzyl, N-phenylsulphamoyl and N-methyl-N-phenylsulphamoyl, and wherein said phenyl substituent or any of said substituents which contain a phenyl group may optionally bear one substituent selected from fluoro, chloro, methyl and methoxy, and Ar may optionally bear a further substituent selected from fluoro, chloro, methyl, ethyl, methoxy, ethoxy and trifluoromethyl; and A^{l}, R¹, R², R³, R⁴ and R⁵ have any of the meanings defined hereinbefore or in this section concerning particular compounds of the invention;
(d) Ar is 5-indenyl or5-indanyl which bears a substituent selected from hydroxyimino, methoxyimino, ethoxyimino, acetoxyimino and cyanomethoxyimino; and A¹, R¹, R², R³, R⁴ and R⁵ have any of the meanings defined hereinbefore or in this section concerning particular compounds of the invention;
(e) Ar is 1-hydroxyiminoindan-5-yl; 1-methoxyiminoindan-5-yl, 1-acetoxyiminoindan-5-yl or 1-cyanome- thoxyiminoindan-5-yl; and A^{l}, R¹, R², R³, R⁴ and R⁵ have any of the meanings defined hereinbefore or in this section concerning particular compounds of the invention;
(f) Ar is a 10-membered benzo-fused heterocyclic moiety containing one or two nitrogen heteroatoms and optionally containing a further heteroatom selected from oxygen and sulphur, which heterocyclic moiety may optionally bear one or two oxo or thioxo substituents and up to two further substituents selected from any of those substituents on Ar defined hereinbefore other than oxo or thioxo; and A¹, R¹, R², R³, R⁴ and R⁵ have any of the meanings defined hereinbefore or in this section concerning particular compounds of the invention;
(g)Ar is quinolyl, 1,2-dihydroquinolyl, 1,2,3,4-tetrahydroquinolyl or 2,3-dihydro-4H-1,4-benzoxazinyl which may optionally bear one oxo or thioxo substituent and up to two further substituents selected from fluoro, chloro, methyl, ethyl, methoxy, ethoxy, trifluoromethyl, acetyl, propionyl, phenyl, benzoyl and benzyl, and wherein each phenyl, benzoyl or benzyl substituent may optionally bear a substituent selected from fluoro, chloro, methyl and methoxy; and A¹, R¹, R², R³, R⁴ and R⁵ have any of the meanings defined hereinbefore or in this section concerning particular compounds of the invention;
(h) Ar is 2-oxo-1,2-dihydroquinolinyl, 2-thioxo-1,2-dihydroquinolinyl, 2-oxo-1,2,3,4-tetrahydroquinolinyl, 2-thioxo-1,2,3,4-tetrahydroquinolinyl or 3-oxo-2,3-dihydro-4H-1,4-benzoxazinyl which may optionally bear up to three substituents selected from fluoro, chloro, methyl, ethyl, methoxy, ethoxy, trifluoromethyl, acetyl, propionyl, phenyl, benzoyl and benzyl, and wherein each phenyl, benzoyl or benzyl substituent may optionally bear a substituent selected from fluoro, chloro, methyl and methoxy; and A¹, R¹, R², R³ R⁴ and R⁵ have any of the meanings defined hereinbefore or in this section concerning particular compounds of the invention;
(i) Ar is 2-oxo-1,2-dihydroquinolin-3-yl, 2-oxo-1,2-dihydroquinolin-6-yl, 2-oxo-1,2-dihydroquinolin-7-yl, 3-oxo-2,3-dihydro-4H-1,4-benzoxazin-7-yl or 3-oxo-2,3-dihydro-4H-1,4-benzothiazin-7-yl which may optionally bear up to three substituents selected from fluoro, chloro, methyl, ethyl, methoxy, ethoxy, trifluoromethyl, acetyl, propionyl, phenyl, benzoyl and benzyl, and wherein each phenyl, benzoyl or benzyl substituent may optionally bear a substituent selected from fluoro, chloro, methyl and methoxy; and A¹, R¹, R², R³, R⁴ and R⁵ have any of the meanings defined hereinbefore or in this section concerning particular compounds of the invention;
(j) Ar is a 10-membered benzo-fused heterocyclic moiety selected from chromanyl and 2,3-dihydro-4H-benzothiapyranyl, which heterocyclic moiety bears a substituent selected from hydroxyimino, methoxyimino, ethoxyimino, acetoxyimino and cyanomethoxyimino; and A^{l}, R^{l}, R², R³, R⁴ and R⁵ have any of the meanings defined hereinbefore or in this section concerning particular compounds of the invention;
(k) Ar is 4-hydroxyiminochromanyl, 4-methoxyiminochromanyl, 4-ethoxyiminochromanyl, 4-acetoxyimino- chromanyl or 4-cyanomethoxyiminochromanyl; and A¹, R¹, R², R³, R⁴ and R⁵ have any of the meanings defined hereinbefore or in this section concerning particular compounds of the invention;
(I) A¹ is methylene; and Ar, R¹, R², R³, R⁴ and R⁵ have any of the meanings defined hereinbefore or in this section concerning particular compounds of the invention;
(m) R⁴ is hydrogen and R⁵ is hydrogen, methyl or ethyl, or each of R⁴ and R⁵ is hydrogen; and Ar, R¹, R² and R³ have any of the meanings defined hereinbefore or in this section concerning particular compounds of the invention;
(n) R¹ is (1-4C)alkyl; and Ar, A^{l}, R², R³, R⁴ and R⁵ have any of the meanings defined hereinbefore or in this section concerning particular compounds of the invention; and
(o) R² and R³ togetherform a group of the formula -A²-X-A³- which together with the carbon atom to which A² and A3 are attached define a ring having 5 or 6 ring atoms, wherein each of A² and A3 is independently methylene or ethylene and X is oxy, and which ring may optionally bear one or two substituents selected from methyl, ethyl and methoxy; and Ar, A¹, R¹, R⁴ and R⁵ have any of the meanings defined hereinbefore or in this section concerning particular compounds of the invention.

A preferred compound of the invention comprises an ester derivative of the formula I
wherein Ar is phenyl which bears one substituent selected from tert-butyl, acetamido, N-methylacetamido, hydroxyiminomethyl, 1-hydroxyiminoethyl, methoxyiminoethyl, 1-methoxyiminoethyl, 1-cyanomethoxyiminoethyl, methanesulphonamido, N-methylmethanesulphonamido, benozyl and benzyl, and wherein said benzoyl or benzyl substituent may optionally bear one substituent selected from fluoro and chloro, or Ar is naphth-2-yl which may optionally bear one substituent selected from fluoro, chloro and methyl,
or Ar is 2-oxo-1,2-dihydroquinolinyl, 2-oxo-1,2,3,4-tetrahydroquinolinyl or 3-oxo-2,3-dihydro-4H-1,4-benzoxa- zinyl which may optionally bear one, two or three substituents selected from fluoro, chloro, methyl and ethyl; A¹ is methylene;
R⁴ is hydrogen;
R⁵ is hydrogen, methyl or ethyl;
R¹ is methyl, ethyl or allyl; and
R² and R³ together form a group of the formula -A²⁻X-A³⁻ which together with the carbon atom to which A² and A3 are attached define a ring having 5 or 6 ring atoms, wherein A² is methylene or ethylene, A3 is ethylene and X is oxy, and which ring may optionally bear one or two substituents selected from methyl and ethyl; or a pharmaceutically-acceptable salt thereof.

A further preferred compound of the invention comprises an ester derivative of the formula I
wherein Ar is naphth-2-yi, 4-[(E)-1-hydroxyiminoethyl]phenyl,
4-[(E)-1-methoxyiminoethyl]phenyl, 4-[(E)-1-cyanomethoxyiminoethyl]phenyl, 1-methyl-2-oxo-1,2-dihydro- quinolin-6-yl or
4-methyl-3-oxo-2,3-dihydro-4H-1,4-benzoxazin-7-yl;
A¹ is methylene;
R⁴ is hydrogen;
R⁵ is hydrogen;
R¹ is methyl, ethyl or allyl; and
R² and R³ toether form a group of the formula-A²-X-A³-which together with the carbon atom to which A² and A3 are attached define a ring having 6 ring atoms, wherein each of A² and A3 is ethylene and X is oxy, and which ring may optionally bear one or two methyl substituents;
or a pharmaceutically-acceptable salt thereof.

A further preferred compound of the invention comprises an ester derivative of the formula I
wherein Ar is phenyl which bears one substituent selected from tert-butyl, acetamido, N-methylacetamido, hydroxyiminomethyl, 1-hydroxyiminoethyl, methoxyiminoethyl, 1-methoxyiminoethyl, 1-cyanomethoxyiminoethyl, methanesulphonamido, N-methylmethanesulphonamido, benozyl and benzyl, and wherein said benzoyl or benzyl substituent may optionally bear one substituent selected from fluoro and chloro, or Ar is naphth-2-yl which may optionally bear one substituent selected from fluoro, chloro and methyl,
or Ar is 2-oxo-1,2-dihydroquinolinyl, 2-oxo-1,2,3,4-tetrahydroquinolinyl or 3-oxo-2,3-dihydro-4H-1,4-benzoxa- zinyl which may optionally bear one, two or three substituents selected from fluoro, chloro, methyl and ethyl, or Ar is 4-hydroxyiminochroman-7-yl, 4-methoxyiminochroman-7-yl, 4-acetoxyiminochroman-7-yl or 4-cyano- methoxyiminochroman-7-yl;
A¹ is methylene;
R⁴ is hydrogen;
R⁵ is hydrogen, methyl or ethyl;
R¹ is methyl, ethyl or allyl; and
R² and R³ together form a group of the formula -A²⁻X-A³⁻ which together with the carbon atom to which A² and A3 are attached define a ring having 5 or 6 ring atoms, wherein A² is methylene or ethylene, A3 is ethylene and X is oxy, and which ring may optionally bear one or two substituents selected from methyl and ethyl; or a pharmaceutically-acceptable salt thereof.

A further preferred compound of the invention comprises an ester derivative of the formula I
wherein Ar is naphth-2-yi, 4-[(E)-1-hydroxyiminoethyl]phenyl,
4-[(E)-1-methoxyiminoethyl]phenyl, 4-[(E)-1-cyanomethoxyiminoethyl]phenyl, 1-methyl-2-oxo-1,2-dihydro- quinolin-6-yl,
4-methyl-3-oxo-2,3-dihydro-4H-1,4-benzoxazin-7-yl,
4-[(E)-hydroxyimino]chroman-7-yl, 4-[(E)-methoxyimino]chroman-7-yl,
4-[(E)-acetoxyimino]chroman-7-yl or
4-[(E)-cyanomethoxyimino]chroman-7-yl;
A¹ is methylene;
R⁴ is methylene;
R⁵ is hydrogen;
R¹ is methyl, ethyl or allyl; and
R² and R³ toether form a group of the formula -A²-X-A³- which together with the carbon atom to which A² and A3 are attached define a ring having 6 ring atoms, wherein each of A² and A3 is ethylene and X is oxy, and which ring may optionally bear one or two methyl substituents;
or a pharmaceutically-acceptable salt thereof.

A further preferred compound of the invention comprises an ester derivative of the formula I
wherein Ar is naphth-2-yi, 4-[(E)-1-methoxyiminoethyl]phenyl or 1-methyl-2-oxo-1,2-dihydroquinolin-6-yl; A¹ is methylene;
R⁴ is hydrogen;
R⁵ is hydrogen;
R¹ is methyl; and
R² and R³ together form a group of the formula -A²⁻X-A³⁻ which together with the carbon atom to which A² and A3 are attached define a ring having 6 ring atoms, wherein each of A² and A3 is ethylene and X is oxy, and which ring may optionally bear a methyl substituent alpha to X;
or a pharmaceutically-acceptable salt thereof.

A further compound of the invention comprises an ester derivative of the formula I
wherein Ar is naphth-2-yi, 4-[(E)-l-methoxyiminoethyl]phenyl, 1-methyl-2-oxo-1,2-dihydroquinolin-6-yl, 4-methyl-3-oxo-2,3-dihydro-4H-1,4-benzoxazin-7-yl or 4-[(E)-methoxyimino] chroman-7-yi;
A¹ is methylene;
R⁴ is hydrogen;
R⁵ is hydrogen;
R¹ is methyl; and
R² and R³ together form a group of the formula -A²⁻X-A³⁻ which together with the carbon atom to which A² and A3 are attached define a ring having 6 ring atoms, wherein each of A²and A3 is ethylene and X is oxy, and which ring may optionally bear a methyl substituent alpha to X;
or a pharmaceutically-acceptable salt thereof.

A specific especially preferred compound of the invention is the following compound of the formula I, or a
pharmaceutically-acceptable salt thereof:-
naphth-2-ylmethyl (Z)-3-amino-3-(4-methoxytetrahydropyran-4-yl)prop-2-enoate or4-[(E)-1-methoxyiminoethyl]benzyl (Z)-3-amino-3-(4-methoxytetrahydropyran-4-yl)prop-2-enoate.

A further specific especially preferred compound of the invention is the following compound of the formula I, or a pharmaceutically-acceptable salt thereof:-4-methyl-3-oxo-2,3-dihydro-4H-1,4-benzoxazin-7-ylmethyl (Z)-3-amino-3-(4-methoxytetrahydropyran-4-yl) prop-2-enoate or 4-[(E)-methoxyimino]chroman-7-ylmethyl (Z)-3-amino-3-(4-methoxytetrahydropyran-4-yl) prop-2-enoate.

A compound of the invention comprising an ester derivative of the formula I, or a pharmaceutically-acceptable salt thereof, may be prepared by any process known to be applicable to the preparation of structurally- related compounds. Suitable procedures are provided hereinafter as a further feature of the invention are are illustrated by the following representative examples in which unless otherwise stated, Ar, A^{l}, R⁴, R⁵, R¹, R² and R³ have any of the meanings defined hereinbefore provided that when there is an amino, imino or hydroxy group in Ar, R² or R³ then any such group may optionally be protected by a conventional protecting group which may be removed when so desired by conventional means.

(a) The transesterification of an ester of the formula II wherein R is a (1-4C)alkyl group, with an alcohol of the formula Ar-A^{l-}OH.

The reaction is conveniently performed in the presence of a suitable acid or base. A suitable acid for the reaction is, for example, hydrochloric, sulphuric, phosphoric, trifluoroacetic or 4-toluene-sulphonic acid, or a Lewis Acid such as a boron trihalide, for example boron trifluoride. Alternatively a suitable acid is provided by an inorganic material, for example a clay such as montmorillonite clay. A suitable base for the reaction is, for example, an alkali or alkaline earth metal carbonate, (1-4C)alkoxide, hydroxide or hydride, for example sodium carbonate, potassium carbonate, sodium ethoxide, potassium butoxide, sodium hydroxide, potassium hydroxide, sodium hydride or potassium hydride; or an organometallic base such as a (1-4C)alkyl-lithium, for example n-butyl-lithium.

The reaction is conveniently performed in a suitable inert solvent or diluent, for example, one or more of 1,2-dimethoxyethane, tetrahydrofruan, methylene chloride, carbon tetrachloride, benzene, toluene or a dipolar aprotic solvent such as N,N-dimethylformamide and dimethylsulphoxide. The reaction is conveniently performed at a temperature in the range for example 10 to 150°C, conveniently at or near 80°C.

The starting materials of the formula II may be obtained by standard procedures of organic chemistry. The preparation of such starting materials is described within the accompanying non-limiting Examples. Alternatively necessary starting materials are obtainable by analogous procedures to those illustrated which are within the ordinary skill of an organic chemist.

A suitable protecting group for an amino or imino group is, for example, an acyl group for example a (2-4C)alkanoyl group (especially acetyl), a (1-4C)alkoxycarbonyl group (especially methoxycarbonyl, ethoxycarbonyl or tert-butoxycarbonyl), an arylmethoxycarbonyl group (especially benzyloxycarbonyl) or an aroyl group (especially benzoyl). The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a tert-butoxycarbonyl group may be removed, for example, by treatment with a suitable acid such as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-charcoal.

A suitable protecting group for a hydroxy group is, for example, an acyl group, for example a (2-4C)alkanoyl group (especially acetyl), an aroyl group (especially benzoyl) or an arylmethyl group (especially benzyl). The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-charcoal.

(b) For the production of those compounds of the formula I wherein each of R⁴ and R⁵ is hydrogen, the coupling, preferably in the presence of a suitable catalyst, of a compound of theformula III with a compound of the formula Ar-A^{l-}O.CO-CH₂-Z wherein Z is a displaceable group.

A suitable displaceable group Z is, for example, a halogeno or sulphonyloxy group, for example a fluoro, chloro, bromo, iodo, methanesulphonyloxy or 4-toluenesulphonyloxy group.

The coupling reaction is conveniently performed in a suitable inert solvent or diluent as defined hereinbefore, and at a temperature in the range, for example, 10 to 150°C, conveniently at or near 70°C.

The reaction may conveniently be performed in the presence of a suitable catalyst, for example a ma- tallic catalyst, for example zinc, copper or magnesium.

The starting materials of the formula Ar-A^{l-}O.CO-CH₂-Z may be obtained by standard procedures of organic chemistry. Starting materials of the formula III are obtainable by analogous procedures to those illustrated in the accompanying Examples or by modifications thereto which are within the ordinary skill of an organic chemist.

(c) For the production of those compounds of the formula I wherein X is a sulphinyl or sulphonyl group, the oxidation of a compound of the formula I wherein X is a thio group.

A suitable oxidising agent is, for example, any agent known in the art for the oxidation of thio to sulphinyl and/or sulphonyl, for example, hydrogen peroxide, a peracid (such as 3-chloroperoxybenzoic or peroxyacetic acid), an alkali metal peroxysulphate (such as potassium peroxymonosulphate), chromium trioxide or gaseous oxygen in the presence of platinum. The oxidation is generally carried out under as mild conditions as possible and with the required stoichiometric amount of oxidising agent in order to reduce the risk of over oxidation and damage to other functional groups. In general the reaction is carried out in a suitable solvent or diluent such as methylene chloride, chloroform, acetone, tetrahydrofuran or tert-butyl methyl ether and at a temperature, for example, at or near ambient temperature, that is in the range 15 to 35°C. When a compound carrying a sulphinyl group is required a milder oxidising agent may also be used, for example sodium or potassium met- aperiodate, conveniently in a polar solvent such as acetic acid or ethanol. It will be appreciated that when a compound of the formula I containing a sulphonyl group is required, it may be obtained by oxidation of the corresponding sulphinyl compound as well as of the corresponding thio compound.

When a pharmaceutically-acceptable salt of a compound of the formula I is required, it may be obtained, for example, by reaction of said compound with a suitable acid or base using a conventional procedure. When an optically active form of a compound of the formula I is required, it may be obtained by carrying out one of the aforesaid procedures using an optically active starting material, or by resolution of a racemic form of said compound using a conventional procedure.

As stated previously, the compounds of the formula I are inhibitors of the enzyme 5-LO. The effects of this inhibition may be demonstrated using one or more of the standard procedures set out below:-
a) An in vitro assay system involving incubating a test compound with heparinised human blood, prior to challenge with the calcium ionophore A23187 and then indirectly measuring the inhibitory effects on 5-LO by assaying the amount of LTB₄ using specific radioimmunoassays described by Carey and Forder (F. Carey and R.A. Forder, Prostaglandins, Leukotrienes Med., 1986, 22, 57; Prostaglandins, 1984, 28, 666; Brit. J. Pharmacol. 1985, 84, 34P) which involve the use of a protein-LTB₄ conjugate produced using the procedure of Young et alia (Prostaglandins, 1983, 26(4), 605-613). The effects of a test compound on the enzyme cyclooxygenase (which is involved in the alternative metabolic pathway for arachidonic acid and gives rise to prostaglandins, thromboxanes and related metabolites) may be measured at the same time using the specific radioimmunoassay for thromboxane B₂(TxB₂) described by Carey and Forder (see above). This test provides an indication of the effects of a test compound against 5-LO and also cyclooxygenase in the presence of blood cells and proteins. It permits the selectivity of the inhibitory effect on 5-LO or cyclooxygenase to be assessed.
b) An ex vivo assay system, which is a variation of test a) above, involving administration to a group of rats of a test compound (usually orally as the suspension produced when a solution of the test compound in dimethylsulphoxide is added to carboxymethylcellulose), blood collection, heparinisation, challenge with A23187 and radioimmunoassay of LTB₄ and TxB₂. This test provides an indication of the bioavailability of a test compound as an inhibitor of 5-LO or cyclooxygenase.
c) An in vivo system involving measuring the effects of a test compound administered orally against the liberation of LTB₄ induced by zymosan within an air pouch generated within the subcutaneous tissue of the back of male rats. The rats are anaesthetised and air pouches are formed by the injection of sterile air (20ml). Afurther injection of air (1 10ml) is similarly given after 3 days. At 6 days after the initial air injection the test compound is administered (usually orally as the suspension produced when a solution of the test compound in dimethylsulphoxide is added to hydroxypropylmethylcellulose), followed by the intrapouch injection of zymosan (imi of a 1% suspension in physiological saline). After 3 hours the rats are killed, the air pouches are lavaged with physiological saline, and the specific radioimmunoassay described above is used to assay LTB₄ in the washings. This test provides an indication of inhibitory effects against 5-LO in an inflammatory milieu.

Although the pharmacological properties of the compounds of the formula I vary with structural changes as expected, in general compounds of the formula I possess 5-LO inhibitory effects at the following concentrations or doses in at least one of the above tests a)-c):-
Test a): IC₅₀ (LTB₄) in the range, for example, 0.01-40pM IC₅₀ (TxB₂) in the range, for example, 40-200µM;
Test b): oral ED₅₀(LTB₄) in the range, for example, 0.1-100mg/kg;
Test c): oral ED₅₀(LTB₄) in the range, for example, 0.1-100mg/kg.

No overt toxicity or other untoward effects are present in tests b) and/or c) when compounds of the formula I are administered at several multiples of their minimum inhibitory dose or concentration.

Thus, by way of example, the compound naphth-2-ylmethyl g) -3-amino-3- (4-methoxytetrahydropyran-4-yl)prop-2-enoate has an IC₅₀ of 0.07µM against LTB₄ in test (a); the compound 4-[(F)-1-methoxy- iminoe- thyl]benzyl (Z)-3-amino-3-(4-methoxytetrahydropyran-4-yl)prop-2-enoate has an IC₅₀ of 9.36µM against LTB₄ in test (a); and the compound 4-[(E)-methoxyimino]chroman-7-ylmethyl (Z)-3-amino-3-(4-methoxytetrahydropyran-4-yl)prop-2-enoate has an IC₅₀ of 0.23wM against LTB₄ in test (a) and an IC₅₀ of approximately 1.5 mg/kg against LTB₄ in test (c).

These compounds are examples of compounds of the invention which show selective inhibitory properties for 5-LO as opposed to cyclooxygenase, which selective properties are expected to impart improved therapeutic properties, for example, a reduction in or freedom from the gastrointestinal side-effects frequently associated with cyclooxygenase inhibitors such as indomethacin.

According to a further feature of the invention there is provided a pharmaceutical composition which comprises an ester derivative of the formula I, or a pharmaceutically-acceptable salt thereof, in association with a pharmaceutically-acceptable diluent or carrier.

The composition may be in a form suitable for oral use, for example a tablet, capsule, aqueous or oily solution, suspension or emulsion; for topical use, for example a cream, ointment, gel or aqueous or oily solution or suspension; for nasal use, for example a snuff, nasal spray or nasal drops; for vaginal or rectal use, for example a suppository; for administration by inhalation, for example as a finely divided powder such as a dry powder, a microcrystalline form ora liquid aerosol; for sub-lingual or buccal use, for example a tablet or capsule; or for parenteral use (including intravenous, subcutaneous, intramuscular, intravascular or infusion), for example a sterile aqueous or oily solution or suspension. In general the above compositions may be prepared in a conventional manner using conventional excipients.

The amount of active ingredient (that is an ester derivative of the formula I, or a pharmaceutically-acceptable salt thereof) that is combined with one or more excipients to produce a single dosage form will necessarily vary depending upon the host treated and the particular route of administration. For example, a formulation intended for oral administration to humans will generally contain, for example, from 0.5 mg to 2g of active agent compounded with an appropriate and convenient amount of excipients which may vary from about 5 to about 98 percent by weight of the total composition. Dosage unit forms will generally contain about 1 mg to about 500 mg of an active ingredient.

According to a further feature of the invention there is provided an ester derivative of the formula I, or a pharmaceutically-acceptable salt thereof, for use in a method of treatment of the human or animal body by therapy.

The invention also includes a method of treating a disease or medical condition mediated alone or in part by one or more leukotrienes which comprises administering to a warm-blooded animal requiring such treatment an effective amount of an active ingredient as defined above. The invention also provides the use of such an active ingredient in the production of a new medicament for use in a leukotriene mediated disease or medical condition.

The size of the dose for therapeutic or prophylactic purposes of a compound of the formula I will naturally vary according to the nature and severity of the conditions, the age and sex of the animal or patient and the route of administration, according to well known principles of medicine. As mentioned above, compounds of the formula I are useful in treating those allergic and inflammatory conditions which are due alone or in part to the effects of the metabolites of arachidonic acid arising by the linear (5-LO catalysed) pathway and in par- ticularthe leukotrienes, the production of which is mediated by 5-LO. As previously mentioned, such conditions include, for example, asthmatic conditions, allergic reactions, allergic rhinitis, allergic shock, psoriasis, atopic dermatitis, cardiovascular and cerebrovascular disorders of an inflammatory nature, arthritic and inflammatory joint disease, and inflammatory bowel diseases.

In using a compound of the formula I for therapeutic or prophylactic purposes it will generally be administered so that a daily dose in the range, for example, 0.5mg to 75mg per kg body weight is received, given if required in divided doses. In general lower doses will be administered when a parenteral route is employed. Thus, for example, for intravenous administration, a dose in the range, for example, 0.5mg to 30 mg per kg body weight will generally be used. Similarly, for administration by inhalation, a dose in the range, for example, 0.5 mg to 25 mg per kg body weight will be used.

Although the compounds of the formula I are primarily of value as therapeutic agents for use in warm-blooded animals (including man), they are also useful whenever it is required to inhibit the enzyme 5-LO. Thus, they are useful as pharmacological standards for use in the development of new biological tests and in the search for new pharmacological agents.

By virtue of their effects on leukotriene production, the compounds of the formula I have certain cytoprotective effects, for example they are useful in reducing or suppressing certain of the adverse gastrointestinal effects of the cyclooxygenase inhibitory non-steroidal anti-inflammatory agents (NSAIA), such as indomethacin, acetylsalicylic acid, ibuprofen, sulindac, tolmetin and piroxicam. Furthermore, co-administration of a 5-LO inhibitor of the formula I with a NSAIA can result in a reduction in the quantity of the latter agent needed to produce a therapeutic effect, thereby reducing the likelihood of adverse side-effects. According to a further feature of the invention there is provided a pharmaceutical composition which comprises an ester derivative of the formula I, or a pharmaceutically-acceptable salt thereof as defined hereinbefore, in conjunction or admixture with a cyclooxygenase inhibitory non-steroidal anti-inflammatory agent (such as those mentioned above), and a pharmaceutically-acceptable diluent or carrier.

The cytoprotective effects of the compounds of the formula I may be demonstrated, for example in a standard laboratory model which assesses protection against indomethacin-induced or ethanol-induced ulceration in the gastrointestinal tract of rats.

The compositions of the invention may in addition contain one or more therapeutic or prophylactic agents known to be of value for the disease under treatment. Thus, for example a known platelet aggregation inhibitor, hypolipidemic agent, anti-hypertensive agent, beta-adrenergic blocker or a vasodilator may usefully also be present in a pharmaceutical composition of the invention for use in treating a heart or vascular disease or condition. Similarly, by way of example, an anti-histamine, steroid (such as beclomethasone dipropionate), sodium cromoglycate, phosphodiesterase inhibitor or a beta-adrenergic stimulant may usefully also be present in a pharmaceutical composition of the invention for use in treating a pulmonary disease or condition.

The invention will now be illustrated in the following non-limiting Examples in which, unless otherwise stated:-
(i) evaporations were carried out by rotary evaporation in vacuo and work-up procedures were carried out after removal of residual solids by filtration;
(ii) operations were carried out at room temperature, that is in the range 18-25°C and under an atmosphere of an inert gas such as argon;
(iii) column chromatography (by the flash procedure) and medium pressure liquid chromatography (MPLC) were performed on Merck Kieselgel silica (Art. 9385) or Merck Lichroprep RP-18 (Art. 9303) reversed-phase silica obtained from E. Merck, Darmstadt, W. Germany;
(iv) yields are given for illustration only and are not necessarily the maximum attainable;
(v) the end-products of the formula I have satisfactory microanalyses and their structures were confirmed by nuclear magnetic resonance (NMR) and mass spectral techniques; unless otherwise stated, CDCI₃ solutions of the end-products of the formula I were used for the determination of NMR spectral data, chemical shift values were measured on the delta scale; the following abbreviations have been used: s, singlet; d, doublet; t, triplet; m, multiplet;
(vi) intermediates were not generally fully characterised and purity was assessed by thin layer chromatographic, infra-red (IR) or NMR analysis;
(vii) melting points are uncorrected and were determined using a Mettler SP62 automatic melting point apparatus or an oil-bath apparatus; melting points for the end-products of the formula I were determined after crystallisation from a conventional organic solvent such as ethanol, methanol, acetone, ether or hexane, alone or in admixture; and
(viii) the following abbreviations have been used:-
   THF tetrahydrofuran;
   DMF N,N-dimethylformamide.

### Example 1

1,2-Dibromoethane (0.1 ml) and 4-cyano-4-methoxytetrahydropyran (0.21 g) were added in turn to a stirred suspension of zinc powder (0.486 g) in THF (20 ml) which was heated to reflux. A solution of naphth-2-ylmethyl bromoacetate (0.837 g) in THF (5 ml) was added dropwise over a period of 1 hour whilst the mixture was stirred and maintained at reflux. The mixture was stirred at reflux for a further 2 hours. The mixture was cooled to ambient temperature and neutralised by the addition of a saturated aqueous potassium carbonate solution. The mixture was stirred for 30 minutes, filtered and evaporated. The residue was purified by column chromatography using a 3:2 mixture of petroleum ether (b.p. 40-60°C) and diethyl ether as eluent. There was thus obtained naphth-2-ylmethyl (Z)-3-amino-3-(4-methoxytetrahydropyran-4-yi)prop-2-enoate (0.2 g, 40%), m.p. 110-112°C.

NMR Spectrum 1.7-1.9 (m, 4H), 3.06 (s, 3H), 3.5-3.7 (m, 4H), 4.6 (s, 1 H), 5.22 (s, 2H), 6.9 (broad s, 1 H), 7.4-7.6 (m, 3H), 7.9-8.0 (m, 5H).

The naphth-2-ylmethyl bromoacetate used as a starting material was obtained as follows:-
2-Bromoacetyl bromide (1.9 ml) was added dropwise to a mixture of 2-naphthalenemethanol (3.16 g), triethylamine (2.8 ml) and methylene chloride (100 ml) which had been cooled to 0°C. The mixture was stirred for 2 hours and allowed to warm to ambient temperature. The mixture was evaporated and the residue was purified by column chromatography using a 7:3 mixture of petroleum ether (b.p. 40-60°C) and methylene chloride as eluent. There was thus obtained the required starting material (3 g, 54%) as a solid.

The 4-cyano-4-methoxytetrahydropyran used as a starting material was obtained as follows:-

A mixture of tetrahydropyran-4-one (5 g), acetone dimethyl acetal (7.4 ml), 4-toluenesulphonic acid (0.01 g) and methanol (5 ml) was stirred and heated to 75°C for 1 hour, during which time the solvent was distilled from the reaction mixture. The residue was neutralised by the addition of a methanolic solution of sodium methoxide. The resultant mixture was purified by distillation under reduced pressure. There was thus obtained tetrahydropyran-4-one dimethyl acetal (5.9 g, 80%), b.p. 80°C at 20 mm of mercury.

A portion (2.25 g) of the material so obtained was-added dropwise to a stirred mixture of titanium tetrachloride (1.75 ml) and methylene chloride (50 ml) which had been cooled to -78°C. Tert-butyl isocyanide (1.9 ml) was added the mixture was allowed to warm to 0°C over 1 hour. The mixture was partitioned between diethyl etherand a saturated aqueous sodium carbonate solution. The organic phase was washed with brine, dried (MgS0₄) and evaporated. The residue was purified by column chromatography using a 4:1 mixture of petroleum ether (b.p. 40-60°C) and diethyl ether as eluent. There was thus obtained the required starting material as an oil (1.24 g, 60%).

### Example 2

1,2-Dibromoethane (0.1 ml) and 4-cyano-4-methoxytetrahydropyran (0.14 g) were added in turn to a stirred suspension of zinc powder (0.325 g) in THF (20 ml) which was heated to reflux. A solution of 4-[(F)-1-methoxyiminoethyl]benzyl bromoacetate (0.57 g) in THF (5 ml) was added dropwise over a period of 90 minutes whilst the mixture was stirred and maintained at reflux. The mixture was heated to reflux for a further hour. The mixture was cooled to ambient temperature and neutralised by the addition of a saturated aqueous potassium carbonate solution. The mixture was stirred at ambient temperature for 30 minutes, filtered and evaporated. The residue was purified by column chromatography using a 3:2 mixture of petroleum ether (b.p. 40-60°C) and diethyl ether as eluent. There was thus obtained 4-[(E)-1-methoxyiminoethyl]benzyl (Z)-3-amino-3-(4-methoxytetrahydropyran-4-yl)prop-2-enoate (0.094 g, 26%), m.p. 75-77°C.

NMR Spectrum 1.7-1.9 (m, 4H), 2.15 (s, 3H), 3.07 (s, 3H), 3.6-3.8 (m, 4H), 3.92 (s, 3H), 4.63 (s, 1 H), 5.06 (s, 2H), 5.2 (broad hump, 1 H), 7.31 (d, 2H), 7.57 (d, 2H), 7.9 (broad hump, 1 H).

The 4-[(E)-1-methoxyiminoethyl]benzyl bromoacetate used as a starting material was obtained as follows:-
A mixture of 4'-bromoacetophenone (10 g), ethylene glycol (14 ml), trimethyl orthoformate (28 ml), 4-toluenesulphonic acid (0.01 g) and toluene (100 ml) was stirred and heated to 60°C for 3 hours. The mixture was cooled to ambient temperature, washed with a-saturated aqueous sodium bicarbonate solution and with water and evaporated. There was thus obtained 4'-bromoacetophenone ethylene acetal (11 g, 91%).

n-Butyl-lithium (1.5M in hexane, 26 ml) was added dropwise to a stirred solution of4'-bromoacetophenone ethylene acetal (7.3 g) in THF (70 ml) which had been cooled to -78°C. The mixture was stirred at -78°C for 15 minutes. DMF (4.6 ml) was added and the mixture was stirred and allowed to warm to ambient temperature. The mixture was partitioned between diethyl ether and a saturated aqueous ammonium chloride solution. The organic layer was washed with brine, dried (MgS0₄) and evaporated. The residue was purified by column chromatography using methylene chloride as eluent. There was thus obtained 4'-formylacetophenone ethylene acetal (3.5 g, 60%),

Sodium borohydride (2 g) was added portionwise to a stirred solution of the acetal so obtained in a mixture of 1,4-dioxan (50 ml) and ethanol (10 ml). The mixture was stirred at ambient temperature for 2 hours. The mixture was evaporated and the residue was partitioned between ethyl acetate and a saturated aqueous ammonium chloride solution. The organic phase was washed with brine, dried (MgS0₄) and evaporated. The residue was purified by column chromatography using a 9:1 mixture of methylene chloride and ethyl acetate as eluent. There was thus obtained 4'-hydroxymethylacetophenone ethylene acetal (2 g, 58%).

A 2N aqueous hydrochloric acid solution (4 drops) was added to solution of the acetal so obtained in acetone (50 ml) and the mixture was stirred at ambient temperature for 45 minutes. The mixture was evaporated and the residue was purified by column chromatography using a 9:1 mixture of methylene chloride and ethyl acetate as eluent. There was thus obtained 4'-hydroxymethylacetophenone (1.5 g, 98%).

A mixture of a portion (1.23 g) of the acetophenone so obtained, hydroxylamine hydrochloride (3.3 g), sodium acetate (3.9 g), water (2 ml) and 1,4-dioxan (20 ml) was stirred and heated to reflux for 2 hours. The mixture was evaporated and the residue was partitioned between ethyl acetate and water. The organic phase was dried (MgS0₄) and evaporated. The residue was recrystallised from ethyl acetate. There was thus obtained (E)-4'-hydroxymethyiacetophenone oxime (0.827 g, 61%).

Sodium hydride (60% dispersion in mineral oil, 0.2 g) was added to a stirred solution of the oxime so obtained in DMF (5 ml) which had been cooled to 0°C and the mixture was stirred for 10 minutes. Methyl iodide (0.625 ml) was added and the mixture was stirred at ambient temperature for 1 hour. The mixture was partitioned between ethyl acetate and water. The organic phase was washed with brine, dried (MgS0₄) and evaporated. The residue was purified by column chromatography using a 30:1 mixture of methylene chloride and ethyl acetate as eluent. There was thus obtained (E)-4'-hydroxymethylacetophenone oxime Q-methyl-ether (0.426 g, 50%).

After appropriate repetition of the steps above, triethylamine (0.86 ml) was added dropwise to mixture of the oxime Q-methyl ether so obtained (1 g), 2-bromoacetyl bromide (0.54 ml) and methylene chloride (30 ml) which had been cooled to 0°C. The mixture was allowed to warm to ambient temperature and was stirred for 90 minutes. The mixture was evaporated and the residue was purified by column chromatography using a 4:1 mixture of methylene chloride and petroleum ether (b.p. 40-60°C) as eluent. There was thus obtained 4-[(E)-1-methoxyiminoethyl]benzyl bromoacetate (1.15 g, 68%).

### Example 3

Sodium hydride (60% dispersion in mineral oil, 0.045 g) was added to a stirred suspension of 6-hydroxymethyl-1-methyl-1,2-dihydroquinolin-2-one (0.19 g) in benzene (30 ml) and the mixture was stirred at ambient temperature for 20 minutes. Methyl (Z)-3-amino-3-(4-methoxytetrahydropyran-4-yi)prop-2-enoate (0.215 g) was added, followed by the addition of methanol (1 drop). The mixture was stirred and heated to reflux for 2 hours whilst the solvent was distilled from the reaction mixture. The residue was purified by column chromatography using a 9:1 mixture of methylene chloride and ethyl acetate as eluent. There was thus obtained 1-methyl-2-oxo-1,2-dihydroquinolin-6-ylmethyl (Z)-3-amino-3-(4-methoxytetrahydropyran-4-yl)prop-2-enoate (0.15 g, 40%), m.p. 152-154°C.

NMR Spectrum 1.5-1.9 (m, 4H), 3.14 (s, 3H), 3.6-3.9 (m, 4H), 3.72 (s, 3H), 4.7 (s, 1 H), 5.19 (s, 2H), 5.3 (broad s, 1 H), 6.72 (d, 1 H), 7.37 (d, 1 H), 7.6 (m, 2H), 7.68 (d, 1 H), 8.0 (broad s, 1 H).

The methyl (Z)-3-amino-3-(4-methoxytetrahydropyran-4-yl)prop-2-enoate used as a starting material was obtained by the reaction of methyl bromoacetate and 4-cyano-4-methoxytetrahydropyran using an analogous procedure to that described in Example 1. There was thus obtained the required starting material in 69% yield, m.p. 80-84°C.

The 6-hydroxymethyl-1-methyl-1,2-dihydroquinolin-2-one used as a starting material was obtained as follows:-

A mixture of 6-bromomethyl-1-methyl-1,2-dihydroquinolin-2-one (European Patent Application No. 0485875, Example 6 thereof; 2.5 g), water (40 ml), sodium carbonate (2.1 g) and acetone (40 ml) was stirred and heated to reflux for 2 hours. The mixture was evaporated. The residue was triturated underwater. The precipitate was isolated and dried. There was thus obtained 6-hydroxymethyl-1-methyl-1,2-dihydroquinolin-2-one (1.24 g, 66%).

NMR Spectrum (CD₃SOCD₃) 3.63 (s, 3H), 4.59 (s, 2H), 5.25 (m, 1 H), 6.6 (d, 1 H), 7.5-7.75 (m, 3H), 7.9 (d, 1 H).

### Example 4

Using an analogous procedure to that described in Example 3, 7-hydroxymethyl-4-methyl-3-oxo-2,3-di- hydro-4H-1,4-benzoxazine was reacted with methyl (Z)-3-amino-3-(4-methoxytetrahydropyran-4-yl)prop-2- enoate to give 4-methyl-3-oxo-2,3-dihydro-4H-1,4-benzoxazin-7-ylmethyl (Z)-3-amino-3-(4-methoxytetrahydropyran-4-yl)prop-2-enoate in 32% yield, m.p. 118-120°C.

NMR Spectrum 1.7-1.9 (m, 4H), 3.14 (s, 3H), 3.36 (s, 3H), 3.7-3.85 (m, 4H), 4.61 (s, 2H), 4.69 (s, 1H), 5.06 (s, 2H), 6.9-7.1 (m, 3H), 5.2-5.4 (m, 1 H), 7.9-8.1 (m, 1 H).

The 7-hydroxymethyl-4-methyl-3-oxo-2,3-dihydro-4H-1,4-benzoxazine used as a starting material was obtained as follows:-

Sodium borohydride (3 g) was added to a solution of 5-formyl-2-nitrophenol (7 g) in a mixture of 1,4-dioxan (80 ml) and ethanol (16 ml). The mixture was stirred at ambient temperature for 2 hours. The mixture was evaporated and the residue was partitioned between ethyl acetate and 2N aqueous hydrochloric acid. The organic phase was washed with water, dried (MgS0₄) and evaporated. There was thus obtained 5-hydroxymethyl-2-nitrophenol (7 g) which was used without further purification.

Ethyl bromoacetate (5.5 ml) was added to a mixture of the product so obtained, potassium carbonate (6.2 g) and DMF (40 ml) and the mixture was stirred at ambient temperature for 16 hours. The mixture was partitioned between ethyl acetate and water. The organic phase was washed with water, dried (MgS0₄) and evaporated. There was thus obtained ethyl 2-(5-hydroxymethyl-2-nitrophenoxy)acetate (11 g) which was used without further purification.

A mixture of the product so obtained, tert-butyldimethyl silyl chloride (8.4 g), imidazole (6.4 g) and methylene chloride (100 ml) was stirred at ambient temperature for 16 hours. The mixture was washed in turn with 2N aqueous hydrochloric acid, a saturated aqueous sodium bicarbonate solution and water. The organic solution was evaporated and the residue was purified by column chromatography using a 4:1 mixture of methylene chloride and petroleum ether (b.p. 40-60°C) as eluent. There was thus obtained ethyl 2-(5-tert-butyldimethylsilyloxymethyl-2-nitrophenoxy)acetate (11.3 g).

A mixture of the product so obtained, palladium-on-charcoal catalyst (10% w/w, 0.4 g) and methanol (300 ml) was stirred at ambient temperature under an atmosphere of hydrogen for 1 hour. The mixture was filtered and the filtrate was evaporated. There was thus obtained 7-(tert-butyldimethylsilyloxymethyl)-3-oxo-2,3-dihydro-4H-1,4-benzoxazine (7.6 g).

Sodium hydride (50% dispersion in mineral oil, 1.3 g) was added to a stirred mixture of the product so obtained, methyl iodide (3.3 ml) and DMF (50 ml) which had been cooled to 0°C. The mixture was stirred at ambient temperature for hours. The mixture was partitioned between ethyl acetate and water. The organic phase was washed with water, dried (MgS0₄) and evaporated. There was thus obtained 7-(tert- butyldimethylsilyloxymethyl)-4-methyl-3-oxo-2,3-dihydro-4H-1,4-benzoxazine (8 g).

A mixture of the product so obtained, a 40% solution of hydrogen fluoride in water (3 ml) and acetonitrile (50 ml) was stirred at ambient temperature for 1 hour. The mixture was evaporated and the residue was partitioned between ethyl acetate and water. The organic phase was washed with water, dried (MgS0₄) and evaporated. The residue was purified by column chromatography using a 49:1 mixture of methylene chloride and methanol as eluent. There was thus obtained the required starting material (3.1 g).

NMR Spectrum 3.35 (s, 3H), 4.59 (s, 2H), 4.63 (s, 2H), 6.8-7.2 (m, 3H).

### Example 5

Using an analogous procedure to that described in Example 3, (E)-7-hydroxymethyichroman-4-one oxime 0-methyi ether was reacted with methyl (Z)-3-amino-3-(4-methoxytetrahydropyran-4-yl)prop-2-enoate to give 4-[(E)-methoximino]chroman-7-ylmethyl(Z)-3-amino-(4-methoxy-tetrahydropyran-4-yl)prop-2-enoate in 23% yield, m.p. 136-139°C.

NMR Spectrum 1.7-2.0 (m, 4H), 2.8-2.9 (m, 2H), 3.14 (s, 3H), 3.65-3.85 (m, 4H), 3.98 (s, 3H), 4.1-4.25 (m, 2H), 4.71 (s, 1 H), 5.07 (s, 2H), 6.9-7.0 (m, 2H), 7.88 (d, 1 H).

The (E)-7-hydroxymethylchroman-4-one oxime 0-methyi ether used as a starting material was obtained as follows:-
A mixture of 3-bromophenol (8.6 g) and 3-chloropropionyl chloride (4.8 ml) was stirred and heated to 110°C for 45 minutes. The mixture was cooled to 80°C and aluminium chloride (20 g) was added portionwise. The mixture was heated to 90°C for a further 2 hours. The mixture was poured onto a mixture of ice and water and extracted with diethyl ether. The organic phase was washed with brine, dried (MgS0₄) and evaporated. The residue was purified by column chromatography using a 4:1 mixture of petroleum ether and methylene chloride as eluent. There was thus obtained 4-bromo-2-hydroxyphenyl 2-chloroethyl ketone (8.2 g, 63%).
A mixture of the product so obtained and 2N aqueous sodium hydroxide (100 ml) was stirred at ambient temperature. The precipitate was isolated and purified by column chromatography using a 4:1 mixture of petroleum ether and methylene chloride as eluent. There was thus obtained 7-bromochroman-4-one in 39% yield, m.p.95-97°C.

Using analogous procedures to those described in the portion of Example 2 which is concerned with the preparation of starting materials, 7-bromochroman-4-one was converted in turn into:-
7-bromochroman-4-one ethylene acetal in 64% yield;
7-formylchroman-4-one ethylene acetal in 75% yield (the n-butyl-lithium and DMF additions were carried out at a reaction temperature of -110°C);
7-hydroxymethylchroman-4-one ethylene acetal in 65% yield;
7-hydroxymethylchroman-4-one, m.p. 62-64°C, in 78% yield;
(E)-7-hydroxymethylchroman-4-one oxime, m.p. 118-120°C, in 75% yield; and (E)-7-hydroxymethylchroman-4-one oxime O-methyl ether, m.p. 78-80°C, in 78% yield.

### CHEMICAL FORMULAE

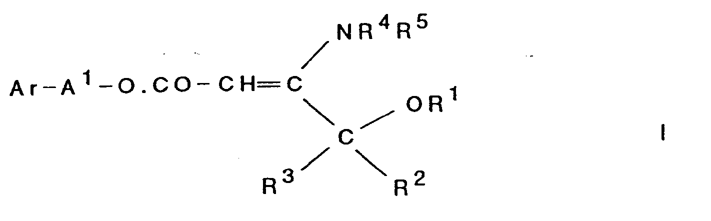

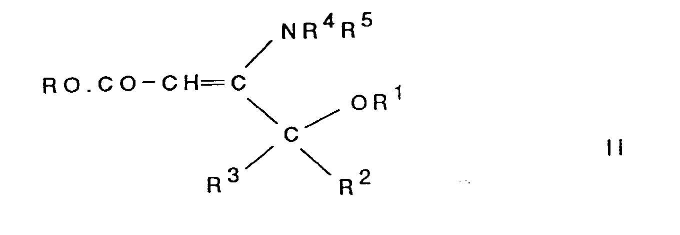

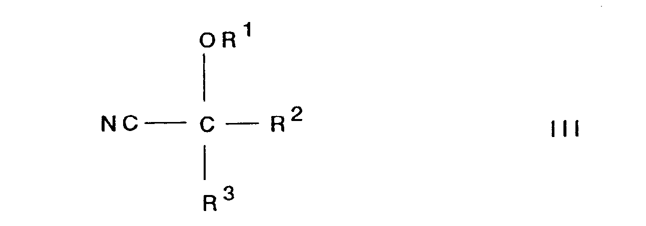

## Claims

1. An ester derivative of the formula I 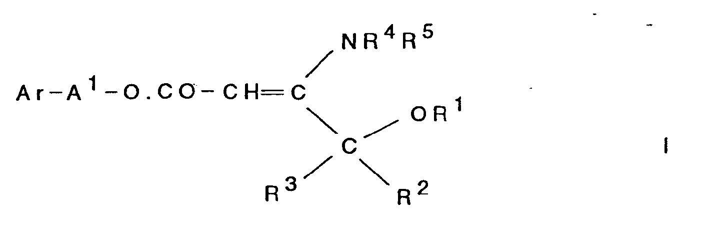
wherein Ar is phenyl, naphthyl, indenyl or indanyl, or a 10-membered bicyclic heterocyclic moiety containing one or two nitrogen heteroatoms and optionally containing a further heteroatom selected from nitrogen, oxygen and sulphur, or a 9-membered or 10-membered bicyclic heterocyclic moiety containing one heteroatom group selected from oxygen, sulphur, sulphinyl, sulphonyl and imino, and Ar may optionally bear up to four substituents selected from halogeno, hydroxy, amino, cyano, formyl, oxo, thioxo, (1-4C)alkyl, (1-4C)alkoxy, fluoro-(1-4C)alkyl, hydroxy-(1-4C)alkyl, (2-4C)alkanoyl, (2-4C)alkanoylamino, N-(1-4C)alkyl-(2-4C)alkanoylamino, hydroxyimino-(1-4C)alkyl, (1-4C)alkoxyimino-(1-4C)alkyl, (2-5C)alka- noyloxyimino-(1-4C)alkyl, cyano-(1-4C)alkoxyimino-(1-4C)alkyl, hydroxyamino(1-4C)alkyl, (1-4C)alkoxyamino-(1-4C)alkyl, N-hydroxyureido-(1-4C)alkyl, N-(1-4C)alkoxyureido-(1-4C)alkyl, N-hydroxy-(2-4C)alkanoylamino-(1-4C)alkyl, N-(1-4C)alkoxy-(2-4C)alkanoylamino-(1-4C)alkyl, (1-6C)alkylideneami- nooxy-(1-4C)alkyl, (1-4C)alkanesulphonamido, N-(1-4C)alkyl-(1-4C)alkanesulphonamido, N-(1-4C)alkyl- sulphamoyl, N,N-di-[(1-4C)alkyl]sulphamoyl, phenyl, benzoyl, benzyl, N-phenylsulphamoyl, N-(1-4C)alkyl-N-phenylsulphamoyl, hydroxyimino, (1-4C)alkoxyimino, (2-5C)alkanoyloxyimino and cyano-(1-4C)al- koxyimino, and wherein said phenyl substituent or any of said substituents which contains a phenyl group may optionally bear a substituent selected from halogeno, (1-4C)alkyl and (1-4C)alkoxy;
A¹ is a direct link to the oxygen atom of the ester group or A¹ is (1-3C)alkylene;
R⁴ is hydrogen or (1-4C)alkyl;
R⁵ is hydrogen or (1-4C)alkyl;
R¹ is (1-4C)alkyl, (3-4C)alkenyl or (3-4C)alkynyl; and
R² and R³ together form a group of the formula -A²⁻X-A³⁻ which together with the carbon atom to which A² and A3 are attached define a ring having 5 or 6 ring atoms, wherein each of A² and A3 is independently (1-3C)alkylene and X is oxy, thio, sulphinyl, sulphonyl or imino, and which ring may optionally bear one or two substituents selected from hydroxy, (1-4C)alkyl and (1-4C)alkoxy;
or a pharmaceutically-acceptable salt thereof.

2. An ester derivative of the formula I as claimed in claim 1 wherein Ar is phenyl which bears one substituent selected from tert-butyl, acetamido, N-methylacetamido, hydroxyiminomethyl, 1-hydroxyiminoethyl, methoxyiminoethyl, 1-methoxyiminoethyl, 1-cyanomethoxyiminoethyl, methanesulphonamido, N-methylmethanesulphonamido, benozyl and benzyl, and wherein said benzoyl or benzyl substituent may optionally bear one substituent selected from fluoro and chloro, or Ar is naphth-2-yl which may optionally bear one substituent selected from fluoro, chloro and methyl,
or Ar is 2-oxo-1 ,2-dihydroquinolinyl, 2-oxo-1 ,2,3,4-tetrahydroquinolinyl or 3-oxo-2,3-dihydro-4H-1,4-ben- zoxazinyl which may optionally bear one, two or three substituents selected from fluoro, chloro, methyl and ethyl;
A¹ is methylene;
R⁴ is hydrogen;
R⁵ is hydrogen, methyl or ethyl;
R¹ is methyl, ethyl or allyl; and
R² and R³ together form a group of the formula -A²⁻X-A³⁻ which together with the carbon atom to which A² and A3 are attached define a ring having 5 or 6 ring atoms, wherein A² is methylene or ethylene, A3 is ethylene and X is oxy, and which ring may optionally bear one or two substituents selected from methyl and ethyl; or a
pharmaceutically-acceptable salt thereof.

3. An ester derivative of the formula I as claimed in claim 1 wherein Ar is phenyl which bears one substituent selected from tert-butyl, acetamido, N-methylacetamido, hydroxyiminomethyl, 1-hydroxyiminoethyl, methoxyiminoethyl, 1-methoxyiminoethyl, 1-cyanomethoxyiminoethyl, methanesulphonamido, N-methylmethanesulphonamido, benozyl and benzyl, and wherein said benzoyl or benzyl substituent may optionally bear one substituent selected from fluoro and chloro, or Ar is naphth-2-yl which may optionally bear one substituent selected from fluoro, chloro and methyl,
or Ar is 2-oxo-1 ,2-dihydroquinolinyl, 2-oxo-1,2,3,4-tetrahydroquinolinyl or 3-oxo-2,3-dihydro-4H-1,4-ben- zoxazinyl which may optionally bear one, two or three substituents selected from fluoro, chloro, methyl and ethyl,
or Ar is 4-hydroxyiminochroman-7-yl, 4-methoxyiminochroman-7-yl, 4-acetoxyiminochroman-7-yl or4-cy- anomethoxyiminochroman-7-yl;
A¹ is methylene;
R⁴ is hydrogen;
R⁵ is hydrogen, methyl or ethyl;
R¹ is methyl, ethyl or allyl; and
R² and R³ together form a group of the formula -A²⁻X-A^{3 -} which together with the carbon atom to which A² and A3 are attached define a ring having 5 or 6 ring atoms, wherein A² is methylene or ethylene, A3 is ethylene and X is oxy, and which ring may optionally bear one or two substituents selected from methyl and ethyl; or a
pharmaceutically-acceptable salt thereof.

4. An ester derivative of the formula I as claimed in claim 1 wherein Ar is naphth-2-yl, 4-[(E)-1-hydroxyiminoethyl]phenyl,
4-[(E)-1-methoxyiminoethyl]phenyl, 4-[(E)-1-cyanomethoxyiminoethyl]phenyl, 1-methyl-2-oxo-1,2-dihy- droquinolin-6-yl,
4-methyl-3-oxo-2,3-dihydro-4H-1,4-benzoxazin-7-yl,
4-[(E)-hydroxyimino]chroman-7-yl, 4-[(E)-methoxyimino]chroman-7-yl, 4-[(E)-acetoxyimino]chroman-7-yl or
4-[(E) -cyanomethoxyimino]chroman-7-yl;
A¹ is methylene;
R⁴ is hydrogen;
R⁵ is hydrogen;
R¹ is methyl, ethyl or allyl; and
R² and R³ toetherform a group of the formula -A²⁻X-A³ which together with the carbon atom to which A² and A3 are attached define a ring having 6 ring atoms, wherein each of A² and A3 is ethylene and X is oxy, and which ring may optionally bear one or two methyl substituents;
or a pharmaceutically-acceptable salt thereof.

5. An ester derivative of the formula I as-claimed in claim 1 wherein Ar is naphth-2-yl, 4-[(E)-1-methoxyiminoethyl]phenyl, 1-methyl-2-oxo-1,2-dihydroquinolin-6-yl, 4-methyl-3-oxo-2,3-dihydro-4H-1,4-benzoxa- zin-7-yl or 4-[(E)-methoxyimino]chroman-7-yl;
A¹ is methylene;
R⁴ is hydrogen;
R⁵ is hydrogen;
R¹ is methyl; and
R² and R³ together form a group of the formula -A²⁻X-A³⁻ which together with the carbon atom to which A² and A3 are attached define a ring having 6 ring atoms, wherein each of A² and A3 is ethylene and X is oxy, and which ring may optionally bear a methyl substituent alpha to X;
or a pharmaceutically-acceptable salt thereof.

6. An ester derivative of the formula I, or a pharmaceutically-acceptable salt thereof, selected from:- naphth-2-ylmethyl (Z)-3-amino-3-(4-methoxytetrahydropyran-4-yl)prop-2-enoate and 4-[(E)-1-methoxyiminoethyl]benzyl (Z)-3-amino-3-(4-methoxytetrahydropyran-4-yl)prop-2-enoate.

7. An ester derivative of the formula I, or a pharmaceutically-acceptable salt thereof, selected from:-4-methyl-3-oxo-2,3-dihydro-4H-1,4-benzoxazin-7-ylmethyl (Z)-3-amino-3-(4-methoxytetrahydropyran-4-yl)prop-2-enoate and 4-[(E)-methoxyimino]chroman-7-ylmethyl (Z)-3-amino-3-(4-methoxytetrahydropyran-4-yl)prop-2-enoate.

8. A process for the preparation of an ester derivative of the formula I, or a pharmaceutically-acceptable salt thereof, as claimed in any one of claims 1 to 7 which comprises:-
(a) the transesterification of an ester of the formula II 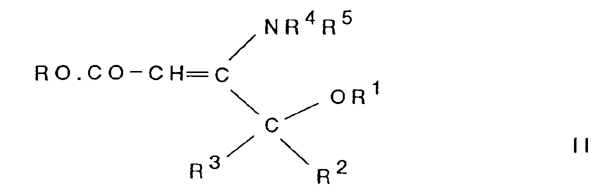
wherein R is a (1-4C)alkyl group, with an alcohol of the formula Ar-A¹-OH;
(b) for the production of those compounds of the formula I wherein each of R⁴ and R⁵ is hydrogen, the coupling of a compound of the formula III 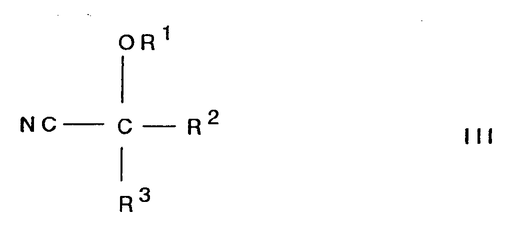
with a compound of the formula Ar-A^{l-}O.CO-CH₂-Z wherein Z is a displaceable group; or
(c) for the production of those compounds of the formula I wherein X is a sulphinyl or sulphonyl group, the oxidation of a compound of the formula I wherein X is a thio group;
and when a pharmaceutically-acceptable salt of a compound of the formula I is required, it may be obtained by reaction of said compound with a suitable acid or base using a conventional procedure, and when an optically active form of a compound of the formula I is required, it may be obtained by carrying out one of the aforesaid procedures using an optically active starting material, or by resolution of a racemic form of said compound using a conventional procedure.

9. A pharmaceutical composition which comprises an ester derivative of the formula I,or a pharmaceutically-acceptable salt thereof, as claimed in any one of claims 1 to 7 in association with a pharmaceutically-acceptable diluent or carrier.

10. The use of an ester derivative of the formula I, or a pharmaceutically-acceptable salt thereof, as claimed in any one of claims 1 to 7 in the production of a new medicament for use in a leukotriene mediated disease or medical condition.
